# EUROPÄISCHE PATENTANMELDUNG

(11) **EP 3 488 872 A1**
(43) Veröffentlichungstag der Anmeldung: **29.05.2019**
(21) Anmeldenummer: 17204065.1
(22) Anmeldetag: 28.11.2017
(51) Int. Cl.: A61L 9/12

(54) **DUFTSTOFFSPENDER**

(71) Anmelder: Wittekind, Herbert, 7500 Silistra (BG)
(72) Erfinder: Wittekind, Herbert, 7500 Silistra (BG)
(74) Vertreter: EGLI-EUROPEAN PATENT ATTORNEYS

(57) **Zusammenfassung**

Die Erfindung betrifft einen Duftstoffspender (20) zum Beduften eines Raumes, mit zumindest einem Duftstoffreservoir (28), welches in einem Gehäuse (22) aufgenommen ist, wobei das Gehäuse (22) als Unterputzgehäuse ausgebildet ist.

## Beschreibung

### Einleitung

Die vorliegende Erfindung betrifft einen Duftstoffspender zum Beduften eines Raums, mit zumindest einem Duftstoffreservoir, welches in einem Gehäuse aufgenommen ist, wobei das Gehäuse als Unterputzgehäuse ausgebildet ist. Bevorzugt umfasst der Duftstoffspender ferner einen Ventilator sowie ein Steuergerät zum Ansteuern des Ventilators.

### Stand der Technik

Aus verschiedensten Gründen kann der Bedarf bestehen, der Raumluft in geschlossenen Räumen Geruchsstoffe zuzusetzen, z.B. weil in diesen Räumen unangenehme Gerüche auftreten können, oder um das Wohlbefinden zu erhöhen. Je nach Anwendungsfall können dabei verschiedene Aspekte in den Vordergrund treten. Zum Beispiel kann es bei der Beduftung von Hotelzimmern von Bedeutung sein, ein möglichst konsistentes Duftprofil zu erzeugen, welches für einen Gast wiedererkennbar ist. Andererseits kann es notwendig sein, das Duftprofil zeitabhängig zu wechseln, so dass beispielsweise gegen Jahresende eine weihnachtliche Duftmischung verwendet wird, während im Frühjahr ein frühjahrshafter Duft ausgebracht werden soll.

Aus dem Stand der Technik sind hierzu vielfältige Lösungen bekannt. Zum einen ist es möglich, Duftstoffe direkt durch Verdampfen in ein Belüftungs- oder Klimatisierungssystem einzubringen. Dies ist jedoch mit dem Nachteil behaftet, dass die Belüftungsleitungen über die Zeit hinweg den Duftstoff annehmen, sodass beispielsweise ein Wechsel des Beduftungsprofils nur schwer möglich ist.

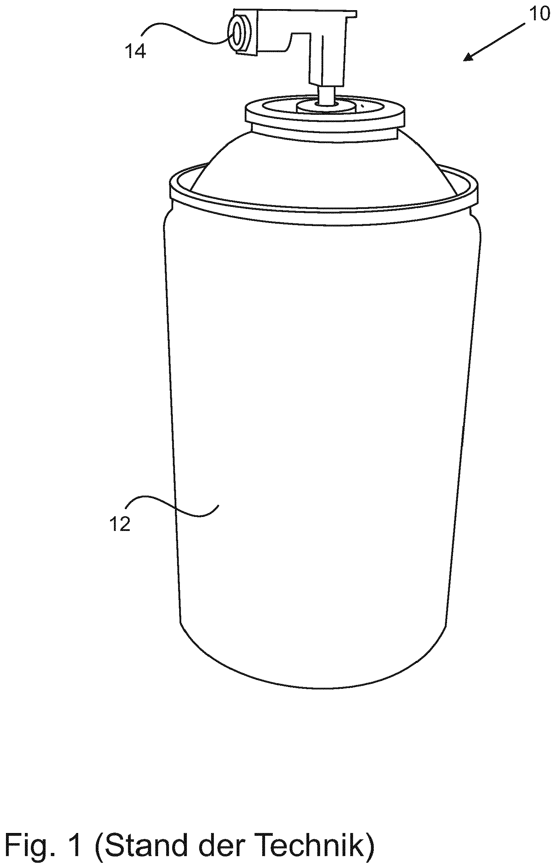

Ferner sind vielfältige Lösungen zur lokalen Anwendung bekannt. Beispielsweise zeigt Fig. 1 eine Beduftungsvorrichtung 10 in Form einer Aerosoldose 12, die ein Duftstoffreservoir und ein Aerosolreservoir umfasst. Aus diesen kann durch Betätigung eines Sprühventils 14 je nach Anwenderwunsch der Duftstoff in gewünschter Menge freigesetzt werden. Dies ist jedoch nur schwer zu kontrollieren und erfordert, dass die im Raum befindlichen Personen selbst aktiv werden.

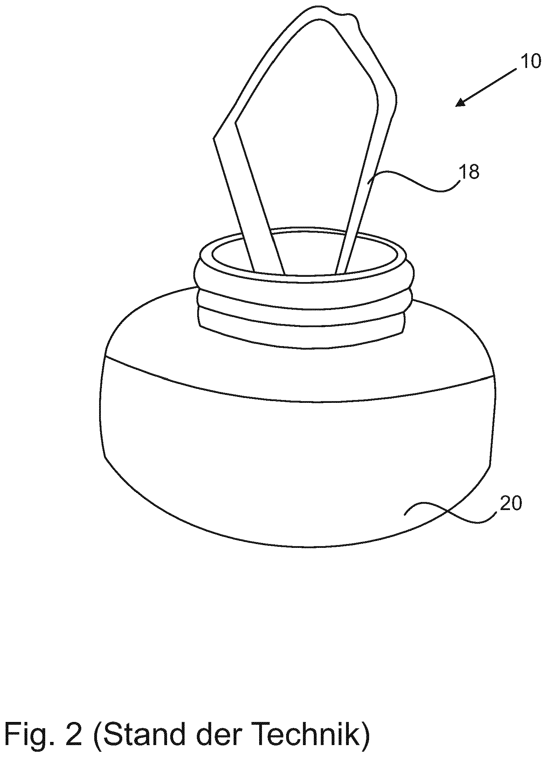

Fig. 2 zeigt einen weiteren Duftstoffspender 10, wie er aus dem Stand der Technik bekannt ist. Hier ist ein flüssiger Duftstoff in einem Reservoir 16 aufgenommen und wird über einen Docht 18 an die Raumluft abgegeben. Es besteht die Möglichkeit der aktiven Steuerung, beispielsweise durch Beheizung des Reservoirs 16 oder des Dochtes 18. Dabei ergibt sich jedoch das Problem, dass die Abgabemenge auch bei einer aktiven Beheizung nur schwer zu kontrollieren ist, wobei zudem flüssige Duftstoffe besonders schnell verbraucht werden, so dass eine derartige Beduftungsvorrichtung beständig gewartet und kontrolliert werden muss.

Duftstoffspender der beschriebenen Art sind zudem in der Regel als freistehende Einzelgeräte ausgeführt und benötigen somit Platz und beeinträchtigen ggf. das ästhetische Erscheinungsbild eines Raumes, in dem sie Anwendung finden.

### Aufgabe der Erfindung

Es ist also die Aufgabe der vorliegenden Erfindung, einen Duftstoffspender bereitzustellen, der kompakt, ästhetisch ansprechend, besonders gut zu steuern und langfristig in seiner Duftstoffabgabe konsistent ist.

### Beschreibung der Erfindung

Die vorliegende Erfindung betrifft einen Duftstoffspender zum Beduften eines Raumes, mit zumindest einem Duftstoffreservoir, welches in einem Gehäuse aufgenommen ist, wobei das Gehäuse als Unterputzgehäuse ausgebildet ist.

Die Verwendung eines Unterputzgehäuses ermöglicht eine besonders einfache verborgene Montage des Duftstoffspenders, beispielsweise gemeinsam mit üblichen, ebenfalls in Unterputzmontage ausgeführten elektrischen Installationen. Damit ergibt sich ein ästhetisch besonders ansprechender Duftstoffspender, der unauffällig überall dort, wo er benötigt wird, angebracht werden kann.

Bei einer bevorzugten Ausführungsform der Erfindung ist das Gehäuse als Gerätedose insbesondere als Zweifachgerätedose oder Dreifachgerätedose, nach DIN 49073 ausgebildet. Bevorzugt ist dabei die Ausführung als Dreifachgerätedose.

Damit kann der Duftstoffspender problemlos in bestehende Elektroinstallationen integriert werden. Auf diese Weise steht zudem unmittelbar die notwendige Versorgungsspannung, bzw. auch Anschlüsse an ggf. vorhandene Steuerleitungen für Hausautomatisierungssysteme oder dgl. zur Verfügung.

Bei einer weiteren bevorzugten Ausführungsform der Erfindung umfasst der Duftstoffspender ferner einen Ventilator, der in fluidischer Verbindung mit dem zumindest einen Duftstoffreservoir steht.

Durch die Verwendung eines Ventilators kann die Menge des abgegebenen Duftstoffes besonders gut kontrolliert werden, weil diese im Wesentlichen proportional zur Luftmasse ist, die das Duftstoffreservoir durchströmt oder umströmt. Gleichzeitig wird so eine zeitabhängige Steuerung der Duftstofffreigabe ermöglicht.

Bei einer weiteren bevorzugten Ausführungsform der Erfindung umfasst der Duftstoffspender ferner einen Gehäusedeckel mit zumindest einer ersten Luftdurchtrittsöffnung, die den Ventilator zumindest teilweise überdeckt, und zumindest einer zweiten Luftdurchtrittsöffnung, die das zumindest eine Duftstoffreservoir zumindest teilweise überdeckt.

Damit wird ein ästhetisch besonders ansprechendes Erscheinungsbild des Duftstoffspenders geschaffen und gleichzeitig ein präzise vorgegebener Luftkanal zwischen den Luftdurchtrittsöffnungen definiert, der die abgegebene Duftstoffmenge besonders gut kontrollierbar macht.

Bei einer weiteren bevorzugten Ausführungsform der Erfindung umfasst der Duftstoffspender ferner ein Netzteil zum Bereitstellen einer Betriebsspannung, vorzugsweise einer Gleichspannung von insbesondere 9 V bis 12 V, für den Duftstoffspender.

Neben der Hauptfunktion, den Ventilator mit einer Betriebsspannung zu versorgen, bringt ein derartiges Netzteil den weiteren Vorteil mit sich, dass der vom Netzteil umfasste Transformator bzw. auch die Leistungselektronik des Netzteils im Betrieb Wärme abgibt und so durch Erwärmung des Duftstoffreservoirs die Duftstofffreigabe befördern kann.

Bei einer weiteren bevorzugten Ausführungsform der Erfindung umfasst der Duftstoffspender ferner ein Steuergerät zum Ansteuern des Ventilators, welches dazu ausgelegt ist, zumindest einen Betriebsparameter des Duftstoffspenders, insbesondere eine Betriebszeit und/oder eine Laufgeschwindigkeit des Ventilators und/oder eine Auswahl aus einer Mehrzahl von Duftstoffreservoirs zu steuern.

Dies ermöglicht eine besonders gute Kontrolle des Duftstoffspenders, sodass sowohl die freigegebene Menge des Duftstoffs, als auch das Zeitprofil der Duftstofffreigabe, sowie das Duftprofil selbst frei und flexibel geändert werden können.

Bei einer weiteren bevorzugten Ausführungsform der Erfindung umfasst das Steuergerät ein Eingabemodul, mittels welchem eine Steuervorgabe für den zumindest einen Betriebsparameter eingebbar ist.

Ein solches Eingabemodul kann beispielsweise in Form einer Anzeige und einer Mehrzahl von Tasten auf dem Gehäusedeckel des Duftstoffspenders verwirklicht sein, sodass ein Benutzer ohne Weiteres jederzeit das gewünschte Beduftungsprofil einstellen kann.

Um die Ästhetik des Duftstoffspenders zu verbessern, ist es auch möglich, derartige Eingabeelemente innerhalb des Gehäuses anzubringen, sodass diese im Normalbetrieb vom Gehäusedeckel verborgen sind.

Bei einer weiteren bevorzugten Ausführungsform der Erfindung umfasst das Steuergerät ein Verbindungsmodul, mittels welchem Daten zwischen dem Steuergerät und einem Hausautomatisierungsbus austauschbar sind.

Dies ermöglicht eine zentrale Steuerung des Duftstoffspenders, was insbesondere in grösseren öffentlichen Gebäuden, wie beispielsweise Hotels, Einkaufscentern, Krankenhäuser, Altenheimen, oder dgl. sinnvoll sein kann, da auf diese Weise die Einstellungen nicht an jedem einzelnen Duftstoffspender geändert werden müssen. Der Duftstoffspender selbst kann über das Verbindungsmodul und den Hausautomatisierungsbus ebenfalls Daten zurücksenden, so dass beispielsweise die Hausverwaltung zentral informiert werden kann, wenn das Duftstoffreservoir eines bestimmten angeschlossenen Duftstoffspenders gewechselt werden muss.

Bei dem Hausautomatisierungsbus kann es sich beispielsweise um Bussysteme wie den europäischen Installationsbus (EIB) nach EN 50090, den Konnex Bus (KNX), den Local Operating Network (LON) Feldbus nach Ansi/EIA 709.X, EIA-852, sowie EN 14908, sowie das Standard Motor Interface (SMI) oder dergleichen handeln.

Bei einer weiteren bevorzugten Ausführungsform der Erfindung umfasst das Steuergerät ein Kommunikationsmodul, mittels welchem Daten zwischen dem Steuergerät und einem Datenverarbeitungssystem außerhalb des Duftstoffspenders drahtlos austauschbar sind.

Auf diese Art und Weise kann beispielsweise über eine W-LAN- oder Bluetooth-Verbindung eine Datenverbindung mit einem mobilen Endgerät, wie beispielsweise einem Smartphone oder einem Laptop oder Tablet hergestellt werden, sodass ein Benutzer unmittelbar Änderungen an den Betriebsparametern des Duftstoffspenders vornehmen kann oder auch über dessen Betriebszustand Informationen abrufen kann.

Eine drahtlose Kommunikation ist ferner auch mit Funkbussystemen möglich, die den Duftstoffspender drahtlos mit einer zentralen Hausautomatisierung verbinden können. Beispielsweise kann hierzu der KNX-RF Standard nach ISO/IEC 14543-3, der ZigBee Standard nach IEE 802.15.4, der IP500 Standard, der HOMEeasy Standard oder dergleichen verwendet werden. Hier treffen die gleichen Vorteile zu, die bereits anhand der kabelgebundenen Hausautomatisierungsbusse oben erläutert wurden.

Bei einer weiteren bevorzugten Ausführungsform der Erfindung umfasst das Steuergerät ein Speichermodul, in welchem ein Steuerprogramm betreffend zumindest einen Betriebsparameter des Duftstoffspenders ablegbar ist.

Auf diese Weise kann zum einen ein Grundprogramm zur Verfügung gestellt werden, welches eine Basisfunktionalität des Duftstoffspenders ohne weiteren Benutzereingriff sicherstellt. Andererseits ist es möglich, einen nichtpermanenten Speicher zu verwenden, in dem vom Benutzer vorgegebene Steuerprogramme abgelegt werden können, sodass diese nicht immer neu eingegeben werden müssen.

Bei einer weiteren bevorzugten Ausführungsform der Erfindung umfasst das Steuergerät einen Bewegungssensor und/oder Geräuschsensor, und ist dazu ausgelegt, bei Feststellen einer Bewegung durch den Bewegungssensor und/oder eines Geräusches durch den Geräuschsensor den Ventilator für einen vorgegebenen Zeitraum zu aktivieren.

Im Gegenzug wird bei Nichtfeststellen einer Bewegung bzw. bei Nichtfeststellen eines Geräusches keine Aktivierung des Ventilators durchgeführt. Hierdurch kann sichergestellt werden, dass die Beduftung nicht nutzloserweise durchgeführt wird, wenn sich ohnehin keine Personen in dem betreffenden Raum aufhalten. Dies ist beispielsweise für die Anwendung in Hotelzimmern besonders sinnvoll.

Bei einer weiteren bevorzugten Ausführungsform der Erfindung umfasst der Duftstoffspender eine Temperiervorrichtung zum Heizen und/oder Kühlen des zumindest einen Duftstoffreservoirs.

Durch die Kontrolle der Temperatur des Duftstoffreservoirs kann die Duftstofffreigabe gesteuert werden. Es ist dabei insbesondere möglich, durch die Umgebungstemperatur bedingte Schwankungen in der freigesetzten Duftstoffmenge auszugleichen, so dass eine besonders konsistente Beduftung verwirklicht wird.

Das Heizen oder Kühlen des zumindest einen Duftstoffreservoirs kann durch direkten Kontakt mit der Temperiervorrichtung erfolgen. Alternativ kann auch die durch den Ventilator zugeführte Luft geheizt oder gekühlt werden. Als Temperiervorrichtung eignen sich beispielsweise Widerstandsheizelemente oder Peltierelemente.

Die Temperiervorrichtung wird bevorzugt ebenfalls von dem Steuergerät gesteuert, bzw. bei Nichtbedarf auch deaktiviert.

Bei einer weiteren bevorzugten Ausführungsform der Erfindung umfasst das zumindest eine Duftstoffreservoir einen porösen luftdurchlässigen Formkörper aus einer Vielzahl von Kunststoffpartikeln, wobei die Kunststoffpartikel Duftstoffe umfassen.

Ein solcher Formkörper kann von dem durch den Ventilator getriebenen Luftstrom durchströmt werden, so dass die Duftstoffe aus den Kunststoffpartikeln freigegeben werden. Im Gegensatz zur Verwendung von Duftstoffreservoirs mit flüssigen Duftstoffen kann so eine besonders sparsame Duftstofffreigabe verwirklicht werden, die zugleich besonders gut zu kontrollieren ist. Insbesondere hat es sich herausgestellt, dass derartige Duftstoffreservoirs nützliche Lebensdauern von drei bis zu ca. sechs Monaten aufweisen, sodass der Duftstoffspender besonders selten gewartet werden muss. Im Gegensatz zu flüssigen Duftstoffen ist auch die Wartung hier besonders einfach, da ein derartiges Duftstoffreservoir einfach und verschmutzungsfrei getauscht werden kann.

Bei einer weiteren bevorzugten Ausführungsform der Erfindung umfasst der Formkörper eine Mehrzahl von Lamellen.

Hierdurch wird eine relative Oberflächenvergrösserung des Formkörpers erreicht, die eine besonders gute Duftstofffreigabe ermöglicht.

Bei einer weiteren bevorzugten Ausführungsform der Erfindung sind die Lamellen zumindest teilweise mit Durchgangsöffnungen versehen.

Auch hierdurch kann eine Oberflächenvergrösserung erzielt werden, die die Freigabe des Duftstoffes verbessert. Durch Einstellung der dem Luftstrom zugänglichen Oberfläche des Kunststoffformkörpers kann in allen Fällen die abgegebene Duftstoffmenge besonders gut kontrolliert werden.

Bei einer weiteren bevorzugten Ausführungsform der Erfindung umfasst der Formkörper eine Mehrzahl von miteinander verbundenen, insbesondere verschmolzenen, zumindest überwiegend kugelförmigen Kunststoffkörpern.

Auch hierdurch wird ein festes Duftstoffreservoir mit besonders guter zugänglicher Oberfläche bereitgestellt, welches zudem besonders einfach zu fertigen ist. Im Gegensatz zur Verwendung von Schüttgut, also losen Kunststoffkörpern, kann ein derartiger Formkörper besonders einfach gewechselt werden, ohne dass es zu Verschmutzungen oder dgl. kommt.

Bei einer weiteren bevorzugten Ausführungsform der Erfindung umfasst das zumindest eine Duftstoffreservoir ein Gehäuse mit einer Mehrzahl von Durchgangsöffnungen, in welchem ein als Schüttgut ausgebildeter Duftstoffträger aufgenommen ist.

Zum Wechsel des Duftstoffes kann hier entweder das Gehäuse mit dem Duftstoff als Ganzes ausgetauscht werden, oder das Gehäuse geöffnet und lediglich der Duftstoffträger gewechselt werden.

Dies ermöglicht die Verwendung von bereits auf dem Markt befindlichen Duftstoffträgern, die nicht speziell an die Form des Duftstoffspenders angepasst werden müssen.

### Kurze Beschreibung der Abbildungen

- Fig. 1:: Figur 1 zeigt ein Ausführungsbeispiel eines Aerosol-Duftstoffspenders nach dem Stand der Technik;
- Fig. 2:: Figur 2 zeigt ein Ausführungsbeispiel eines Verdampfer-Duftstoffspenders nach dem Stand der Technik;

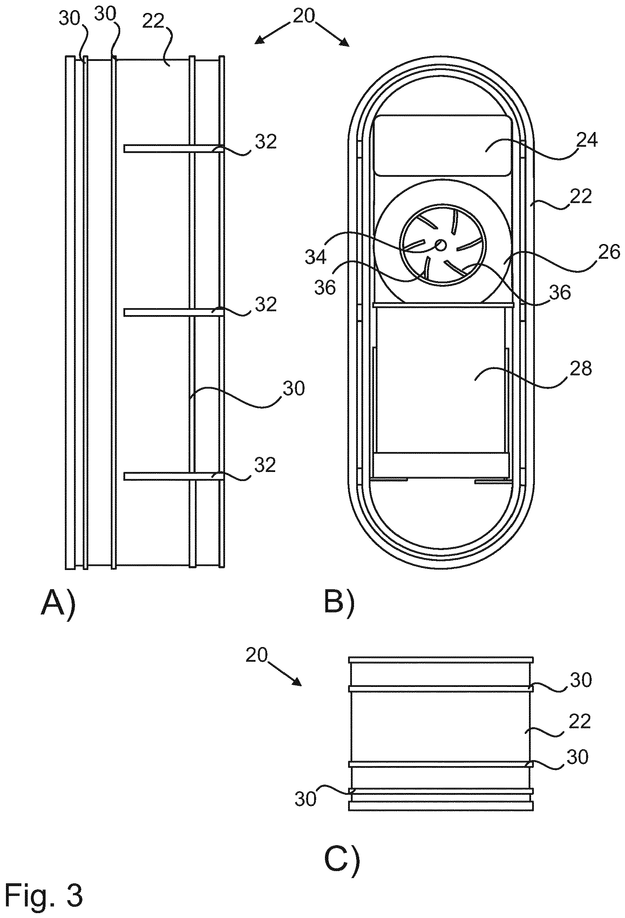

- Fig. 3A:: Figur 3A zeigt eine erste Seitenansicht eines Ausführungsbeispiels eines erfindungsgemässen Duftstoffspenders;
- Fig. 3B:: Figur 3B zeigt eine Draufsicht auf das Ausführungsbeispiel des erfindungsgemässen Duftstoffspenders nach Figur 3A;
- Fig. 3C:: Figur 3C zeigt eine alternative Seitenansicht des erfindungsgemässen Duftstoffspenders nach Fig. 3A;
- Fig. 4:: Figur 4 zeigt eine perspektivische Ansicht eines Gehäuses für ein Ausführungsbeispiel eines erfindungsgemässen Duftstoffspenders;
- Fig. 5:: Figur 5 zeigt eine perspektivische Ansicht eines weiteren Ausführungsbeispiels eines erfindungsgemässen Duftstoffspenders;
- Fig. 6:: Figur 6 zeigt eine perspektivische Ansicht eines weiteren Ausführungsbeispiels eines erfindungsgemässen Duftstoffspenders;
- Fig. 7A:: Figur 7A zeigt eine Seitenansicht eines Gehäusedeckels für ein Ausführungsbeispiel eines erfindungsgemässen Duftstoffspenders;
- Fig. 7B:: Figur 7B zeigt eine Draufsicht auf den Gehäusedeckel nach Fig. 7A;
- Fig. 8:: Figur 8 zeigt ein Ausführungsbeispiel eines erfindungsgemässen Duftstoffspenders ohne Gehäusedeckel in Unterputzmontage;
- Fig. 9:: Figur 9 zeigt den Duftstoffspender nach Fig. 8 mit aufgesetztem Gehäusedeckel;
- Fig. 10:: Figur 10 zeigt ein Ausführungsbeispiel eines Duftstoffreservoirs für ein Ausführungsbeispiel eines erfindungsgemässen Duftstoffspenders in Seitenansicht;
- Fig. 11:: Figur 11 zeigt das Duftstoffreservoir gemäss Fig. 10 in perspektivischer Ansicht von schräg oben;
- Fig. 12:: Figur 12 zeigt ein alternatives Ausführungsbeispiel eines Duftstoffreservoirs für einen erfindungsgemässen Duftstoffspender in perspektivischer Ansicht;
- Fig. 13:: Figur 13 zeigt ein weiteres alternatives Ausführungsbeispiel eines Duftstoffreservoirs für einen erfindungsgemässen Duftstoffspender in perspektivischer Ansicht.

Ein im Ganzen mit 20 bezeichneter Duftstoffspender gemäss der vorliegenden Erfindung umfasst ein Gehäuse 22, welches als normgerechte Gerätedose für den Unterputzeinbau ausgebildet ist. Im Inneren des Gehäuses 22 sind ein Elektronikmodul 24, ein Ventilator 26 und ein Duftstoffreservoir 28 vorgesehen.

Das Gehäuse 22 umfasst Rippen 30, die als Rastelemente zum Verrasten des Gehäuses 22 mit der Einbauöffnung in einer Wand dienen können. Um die Verrastung zu verbessern, sind im Gehäuse 22 ferner Aussparungen 32 vorgesehen, die ein leichtes Federn des Gehäuses 22 ermöglichen und so die Klemmkraft verbessern. Ein solches Gehäuse 22 eignet sich zur Unterputzmontage sowohl in festen Mauerwerk, als auch in nichttragenden Wänden. Bevorzugt ist das Gehäuse 22 dabei als Dreifachgerätedose nach der DIN Norm 49073 ausgebildet. Für kompaktere Duftstoffspender 20 kann jedoch auch eine Zweifachgerätedose verwendet werden.

Das Elektronikmodul 24 umfasst ein Netzteil, welches die in der Unterputzöffnung bereitgestellte Netzspannung von 220 bzw. 110 V in eine Betriebsspannung für den Ventilator 26 umsetzt. Bevorzugt ist dabei die Betriebsspannung eine Gleichspannung von 9 V bis 12 V.

Das Elektronikmodul 24 umfasst ferner ein Steuergerät zum Ansteuern des Ventilators 26, welches Betriebszeiten und Betriebsgeschwindigkeiten des Ventilators 26 nach einem vorgegebenen Programm steuern kann. Das Programm kann dabei in einem permanenten oder nichtpermanenten Speicher des Elektronikmoduls 24 abgelegt sein.

Zum Ändern der Programmparameter kann ferner eine Eingabevorrichtung vorgesehen sein, die eine Mehrzahl von Eingabetasten und ggf. eine Anzeigevorrichtung umfasst. Ferner ist es möglich, dass das Elektronikmodul 24 eine Schnittstelle zum Verbinden des Duftstoffspenders 20 mit einem Hausautomatisierungssystem umfasst. Hierzu können die bereits eingangs genannten standardisierten Hausautomatisierungsbusse oder Hausautomatisierungs-Funkbusse verwendet werden. Auch ein Kommunikationsmodul zur drahtlosen Kommunikation, beispielsweise über W-LAN oder Bluetooth kann im Elektronikmodul 24 vorgesehen sein, so dass die Programmeinstellungen des Steuergeräts beispielsweise über ein Smartphone, ein Tablet, oder einen Laptop modifiziert werden können.

Der Ventilator 26 ist im gezeigten Ausführungsbeispiel als Radiallüfter ausgebildet, der in Richtung seiner Drehachse 34 mittels der Lüfterschaufeln 36 Luft ansaugt und diese radial zur Drehachse 34 in das Duftstoffreservoir 28 befördert, aus dem die Luft mit Duftstoff beladen wieder austritt und an die Raumluft abgegeben wird. Selbstverständlich ist auch die Verwendung anderer Lüftertypen möglich.

Im gezeigten Ausführungsbeispiel liegt lediglich ein einzelnes Duftstoffreservoir 28 vor. Es ist jedoch auch möglich, mehrere Duftstoffreservoirs 28, beispielsweise mit unterschiedlichen Duftstoffen bereitzustellen. Gegebenenfalls sind hierbei dann mehrere Luftkanäle zwischen dem Ventilator 28 und der Mehrzahl von Duftstoffreservoirs 28 notwendig, die beispielsweise über Schaltventile geöffnet oder geschlossen werden können, um ausgewählte einzelne Duftstoffe oder ausgewählte Duftstoffmischungen freizusetzen. Die Schaltventile können dann ebenfalls vom Steuergerät des Elektronikmoduls 24 angesteuert werden.

Es ist dabei vorteilhaft, wenn die Abwärme des Elektronikmoduls 24 in Richtung des Duftstoffreservoirs bzw. in Richtung der vom Lüfter 26 angesaugten Luft abgeben wird, so dass durch die erwärmte Luft die Duftstofffreisetzung aus dem Duftstoffreservoir 28 weiter verbessert wird. Zusätzlich kann jedoch auch noch eine Temperiervorrichtung zum Heizen oder Kühlen des Duftstoffreservoirs 28 vorgesehen sein.

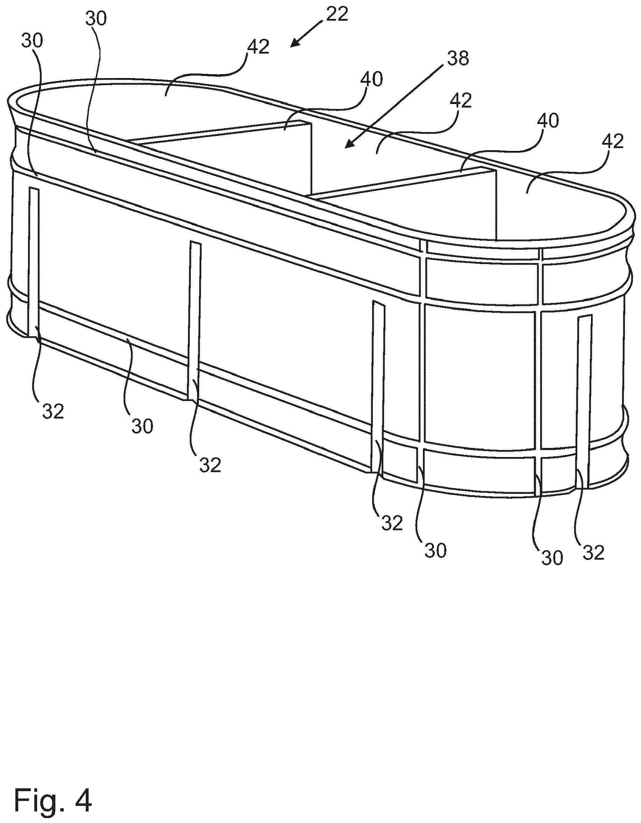

Das Gehäuse 22 ist in perspektivischer Ansicht auch in Fig. 4 dargestellt. Wie bereits geschildert, handelt es sich in diesem Ausführungsbeispiel um eine standardisierte Dreifachgerätedose nach DIN 49073. In der perspektivischen Ansicht ist besonders gut zu erkennen, wie die Längs- und Querrippen 30 und die Aussparungen 32 im Gehäuse 22 angeordnet sind, um eine besonders gute federnde Verrastung des Gehäuses 22 in einer Einbauöffnung im Mauerwerk zu gewährleisten.

Gemäss der oben genannten Norm kann das Gehäuse 22 in seinem Innenraum 38 Trennwände 40 aufweisen, die den Innenraum 38 in drei Kammern 42 aufteilen. Die einzelnen Kammern 42 können dabei jeweils zur Aufnahme des Elektronikmoduls 24, zur Aufnahme des Ventilators 26 und zur Aufnahme des Duftstoffreservoirs 28 genutzt werden. Bevorzugt wird dabei der Ventilator 26 in der mittleren Kammer angeordnet, während die untere Kammer der Aufnahme des Duftstoffreservoirs 28 dient und das Elektronikmodul 24 in der oberen Kammer aufgenommen ist. "Oben" und "unten" beziehen sich dabei jeweils auf die Einbaulage des Duftstoffspenders 20. Es ist jedoch selbstverständlich auch möglich, ein Gehäuse 22 ohne Trennwände 40 zu verwenden.

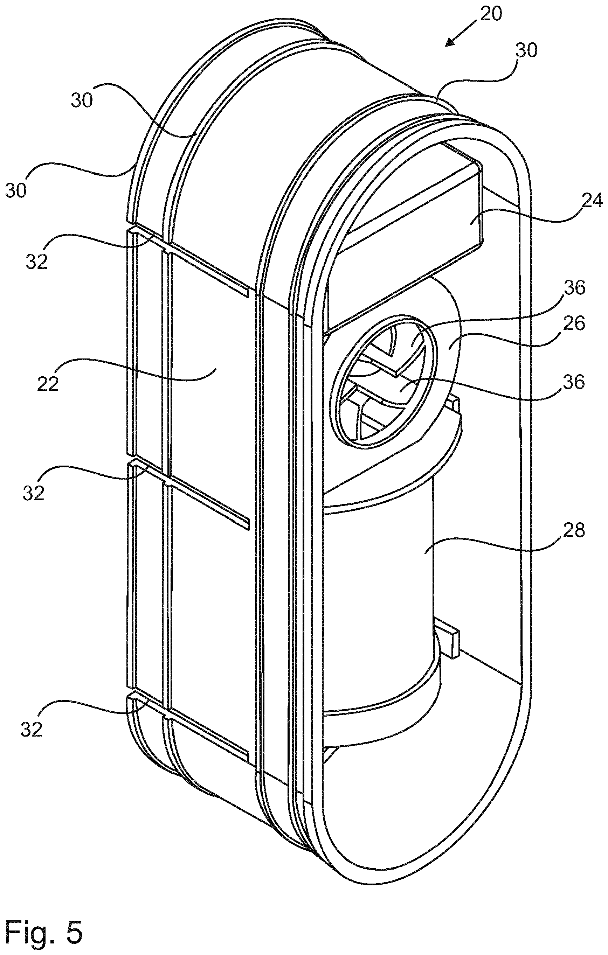

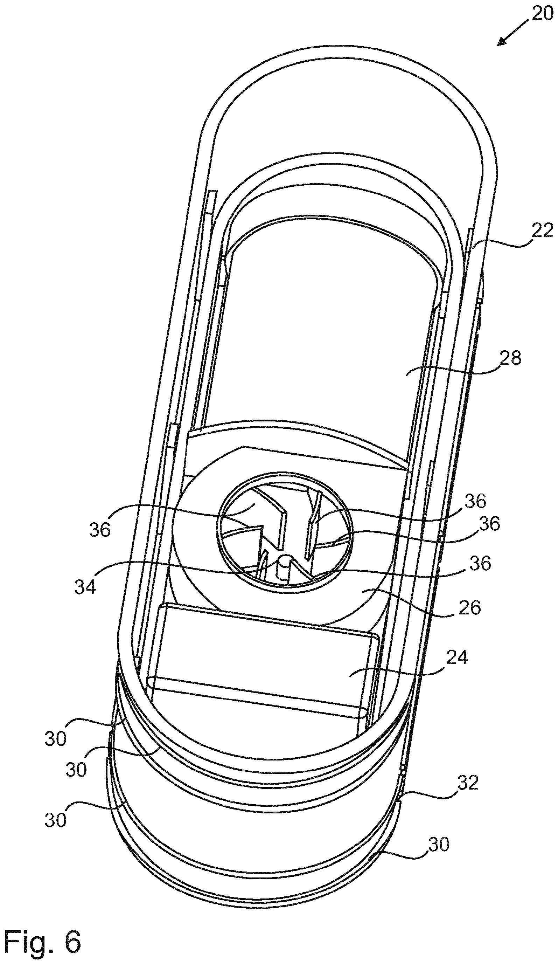

Der Duftstoffspender 20 ist in den Figuren 5 und 6 als Ganzes in perspektivischer Ansicht dargestellt.

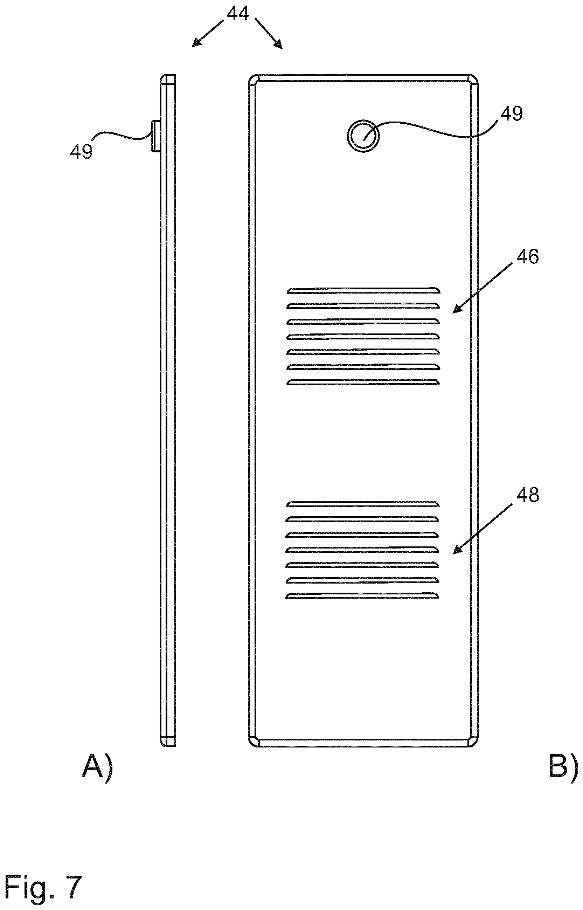

Um dem Duftstoffspender 20 auch nach aussen hin ein ästhetisch ansprechendes Erscheinungsbild zu verleihen, ist zusätzlich ein Gehäusedeckel 44 vorgesehen, der in den Figuren 7A und 7B in Seitenansicht und Draufsicht dargestellt ist. Der Gehäusedeckel 44 weist zwei Sätze von Luftdurchtrittsöffnungen 46, 48 auf. Die Luftdurchtrittsöffnungen 46 überdecken dabei zumindest teilweise den Ventilator 26, während die Luftdurchtrittsöffnungen 48 zumindest teilweise das Duftstoffreservoir 28 überdecken. Damit wird durch die Luftdurchtrittsöffnungen 46 Luft vom Ventilator 26 angesaugt, durch das Duftstoffreservoir 28 befördert und durch die Luftdurchtrittsöffnungen 48 wieder ausgestossen, so dass die Raumluft im gewünschten Masse beduftet wird.

Der Gehäusedeckel 44 verrastet vorzugsweise mit dem Gehäuse 22 des Duftstoffspenders 20. Zum Entriegeln der Verrastung ist ein Entriegelungsknopf 49 vorgesehen, so dass der Gehäusedeckel 44 einfach abgenommen werden kann. Dies ermöglicht einen schnellen und einfachen Austausch des Duftstoffreservoirs 28 und ggf. Zugang zu am Elektronikmodul 24 vorgesehenen Eingabeelementen zur Programmierung des Duftstoffspenders.

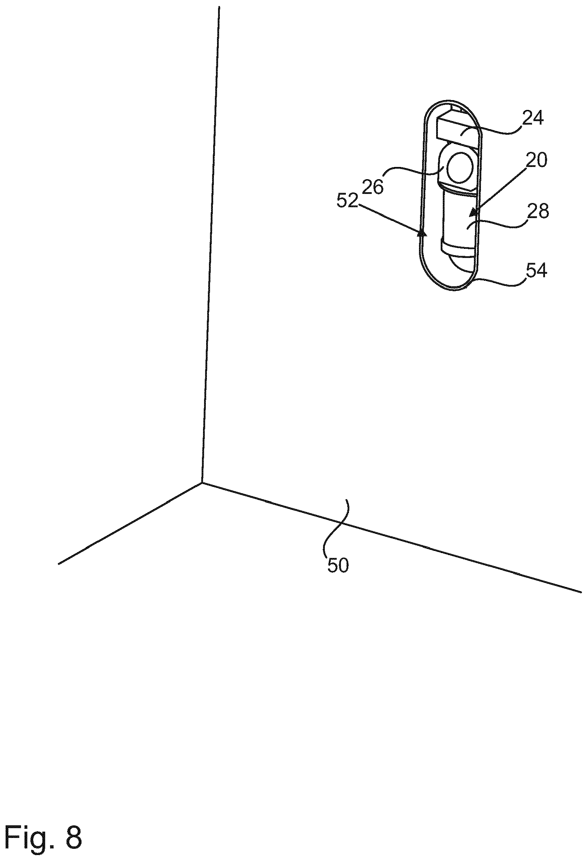

Fig. 8 zeigt den Duftstoffspender 20 in seiner Einbaulage in einer Wand 50, in welcher der Duftstoff 20 in einer Einbauöffnung 52 aufgenommen ist. Eine Oberkante 54 des Gehäuses 22 des Duftstoffspenders 20 schliesst dabei bündig mit der Wand 50 ab, so dass der Duftstoffspender unauffällig und ästhetisch ansprechend angebracht werden kann.

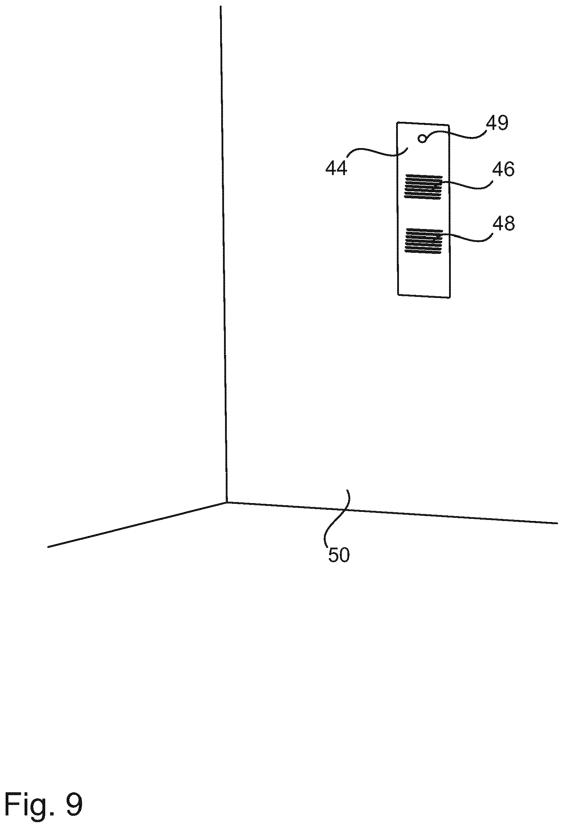

Wie Fig. 9 zeigt, kann nun der Gehäusedeckel 44 aufgesetzt werden, so dass dieser eine bündig mit der Wand 50 abschliessende Oberfläche bildet. Der Gehäusedeckel 44 kann jedoch auch von der Wand abragen bzw. über diese vorstehen.

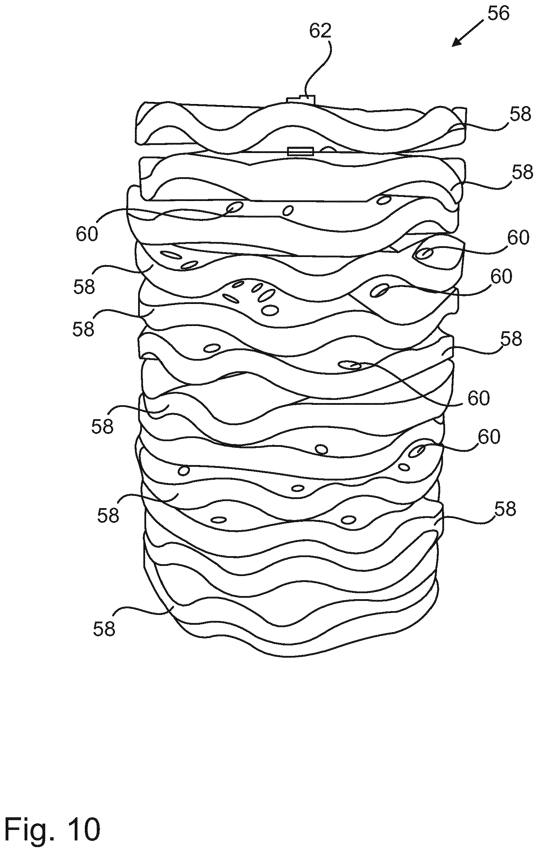

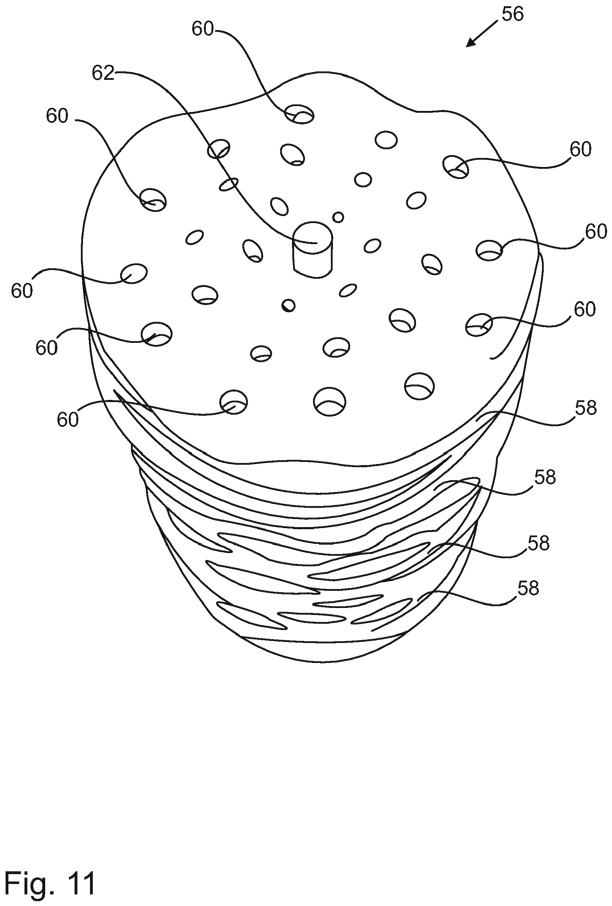

In Fig. 10 ist ein Kunststoffformkörper 56 gezeigt, der als Duftstoffreservoir 28 Anwendung finden kann. Der Kunststoffformkörper 56 besteht aus einem Kunststoff, an dem Duftstoffe gebunden sind, und ist als Stapel von Lamellen 58 gestaltet. Die Lamellen 58 weisen dabei jeweils eine Mehrzahl von Durchgangsöffnungen 60 auf, um die Oberfläche weiter zu vergrössern, den Luftstrom durch den Kunststoffformkörper 56 zu verbessern und damit die Duftstoffabgabe zu erhöhen. Die Lamellen 58 werden dabei von einer Trägerachse 62 gehalten. In Fig. 11 ist dies in einer alternativen perspektivischen Ansicht gezeigt.

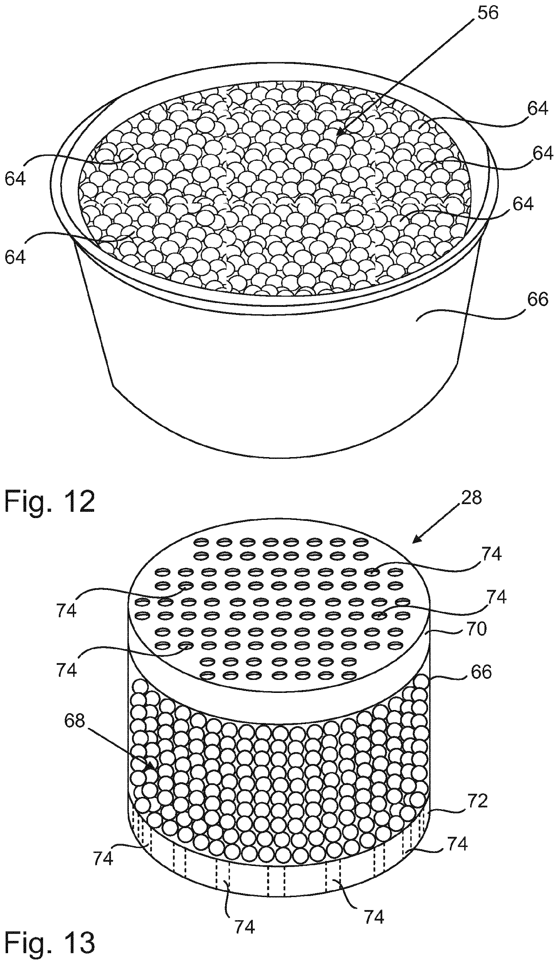

Fig. 12 zeigt ein alternatives Ausführungsbeispiel des Kunststoffformkörpers 56, der hier aus kugelförmigen Kunststoffkörpern 64 aufgebaut ist, die miteinander teilweise verschmolzen und in einem Gehäuse 66 aufgenommen sind.

In beiden gezeigten Beispielen besteht der Kunststoffformkörper 56 aus individuellen Kunststoffpartikeln, die durch Verschmelzen eines Teils der Oberfläche der Kunststoffpartikel miteinander verbunden sind. Hierdurch ergibt sich ein poröser, luftdurchlässiger Formkörper 56, der eine optimale Duftstoffabgabe und Durchströmbarkeit mit Luft gewährleistet. Im Gegensatz zur Verwendung von freien Kunststoffpartikeln in Form von Schüttgut erlaubt diese Form des mit Geruchsstoff beladenen Kunststoffs eine einfache Handhabung des Formkörpers 56 und damit einen einfachen Austausch verbrauchter Duftstoffreservoirs 28.

Dem Formkörper kann fast jede beliebige Form verliehen werden, sodass die Anpassung an die geometrischen Gegebenheiten des Duftstoffspenders 20 besonders einfach ist.

Vorzugsweise weist ein derartiger Formkörper 56 eine Druckfestigkeit von wenigstens 0,1 N/mm² auf, so dass dieser einfach und beschädigungsfrei handzuhaben ist. Besonders bevorzugt ist dabei eine Druckfestigkeit von wenigstens 0,5 N/mm² ganz besonders bevorzugt von wenigstens 1 N/mm² und am meisten bevorzugt von wenigstens 10 N/mm².

Als Trägerkunststoffe für den Duftstoff sind dabei alle Kunststoffe geeignet, die mit Duftstoffen beladen werden können. Bevorzugt weist das Material der Kunststoffpartikel des Formkörpers 56 einen Schmelz- oder Erweichungspunkt im Bereich von 40 bis 125°C, vorzugsweise von 50 bis 100°C, besonders bevorzugt von 60 bis 90°C und ganz besonders bevorzugt von 70 bis 80°C auf.

Beispielsweise können Kunststoffe verwendet werden, die ausgewählt sind aus der Gruppe umfassend Ethylen/Vinylacetat Copolymere, Polyethylen niederer oder hoher Dichte (LDPE, HDPE) oder Gemische derselben, Polypropylen, Polyethylen-Polypropylen-Copolymere, Polyether-Polyamid-Blockcopolymere, Styrol-Butadien-(Block-)Copolymere, Styrol-Isopropen- (Block-)Copolymere, Styrol-Ethylen-Butylen-Copolymere, Acrylnitril-Butadien-Styrol-Copolymere, Acrylnitril-Butadien-Copolymere, Polyetherester, Polyisobuten, Polyisopren, Ethylen-Ethylacrylat-Copolymere, Polyamide, Polycarbonat, Polyester, Polyacrylnitril, Polymethylmethacrylat, Polyurethane und Polyvinylalkohole.

Besonders bevorzugt umfassen die Kunststoffpartikel, die den Formkörper 56 aufbauen, wenigstens ein Ethylen-Vinylacetat-Copolymer. Derartige Copolymere lassen sich einfach mit grossen Mengen von Duftstoffen beladen und die die Duftstoffe enthaltenden Partikel lassen sich dann durch einfaches Erwärmen miteinander zum Formkörper 56 verschmelzen.

Besonders bevorzugt umfassen die Kunststoffpartikel, aus denen der Formkörper 56 geformt ist mindestens 10 Gew.-%, vorzugsweise mindestens 30 Gew.-%, besonders bevorzugt mindestens 70 Gew.-% Ethylen-Vinylacetat-Copolymer, jeweils bezogen auf die Gesamtmasse der in den Partikeln enthaltenen Polymere. Besonders bevorzugt bestehen die Kunststoffpartikel, aus denen der Formkörper 56 gebildet ist, ausschliesslich aus Ethylen-Vinylacetat-Copolymer. Bei den genannten Massenverhältnissen sind die Duftstoffe und etwaige andere enthaltene Zuschläge nicht berücksichtigt.

Das wenigstens eine Ethylen-Vinylacetat-Copolymer weist dabei bevorzugt einen Schmelzindex von 0 bis 400, besonders bevorzugt von 0 bis 100 auf, da sich solche Polymere besonders einfach mit grossen Mengen von Duftstoff beladen und danach zum Formkörper 56 verschmelzen lassen.

Die Kunststoffpartikel, aus denen der Formkörper 56 gebildet ist, können beliebige Formen aufweisen, beispielsweise die Form von Kugeln, Zylindern, Würfeln, Quadern, Linsen, Tabletten, oder dgl. Besonders bevorzugt ist dabei die Kugelform. Der mittlere Durchmesser der verwendeten Kunststoffpartikel beträgt dabei bevorzugt 0,1 mm bis 10 mm, besonders bevorzugt 0,5 mm bis 5 mm und ganz besonders bevorzugt 1 mm bis 3 mm. Die Grösse wird dabei vorzugsweise durch Laserbeugung bestimmt.

Als Geruchsstoffe können dabei alle bekannten Duftstoffe verwendet werden. Beispielsweise kommen dabei synthetische Produkte von Typ der Ester, Ether, Aldehyde, Ketone, Alkohole und Kohlenwasserstoffe in Frage. Geruchsstoffe vom Typ der Ester sind beispielsweise Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmetylphenylglycinat, Allylcyclohexylpropionat, Styrallylpropionat und Benzylsalicylat.

Zu den verwendbaren Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxicitronellal, Lilial und Bourgeonal, zu den Ketonen z.B. die Jonone, X-Isomethylionon und Methylcedrylketon, zu den verwendbaren Alkoholen zählen Anethol, Citronellol, Eugenol, Geraniol, Linalool, Phenylethylalkohol und Terpineol. Verwendbare Kohlenwasserstoffe gehören hauptsächlich in die Gruppe der Terpene wie Limonen und Pinen.

Bevorzugt werden jedoch Mischungen verschiedener Geruchsstoffe verwendet, die gemeinsam eine ansprechende Duftnote erzeugen. Solche Parfümöle können auch natürliche Geruchsstoffgemische enthalten, wie sie aus pflanzlichen Quellen zugänglich sind, wie z.B. Kiefern-, Zitrus-, Jasmin-, Patchouli-, Rosen- oder Ylang-ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Der Gewichtsanteil der Geruchstoffe in den erfindungsgemässen Formkörpern 56 beträgt bevorzugt 1 bis 70 Gew.-%, besonders bevorzugt 10 bis 60 Gew.-%, ganz besonders bevorzugt 20 bis 50 Gew.-% und am meisten bevorzugt 30 bis 40 Gew.-%, jeweils bezogen auf das Gesamtgewicht der Formkörper 56.

Auch wenn es sich bei den Geruchsstoffen um Flüssigkeiten handelt, haben die erfindungsgemässen Formkörper 56 bei solchen Beladungsraten immer noch die Eigenschaften von Festkörpern und lassen sich problemlos handhaben.

Ferner kann der Formkörper 56 weitere Substanzen erhalten, wobei es sich beispielsweise um Substanzen aus der Gruppe der Parfümträger, Farbstoffe, antimikrobieller Wirkstoffe, Herbizide, Fungizide, Antioxidantien oder Korrosionsinhibitoren handeln kann. Diese Substanzen sind dabei bevorzugt Bestandteil der Kunststoffpartikel, die den Formkörper 56 aufbauen. Sie können aber auch zusätzlich zu den Kunststoffpartikeln im Formkörper 56 enthalten sein.

Wie in Fig. 13 gezeigt, kann das Duftstoffreservoir 28 ferner ein Gehäuse 66 umfassen, in dem ein loser, als Schüttgut ausgebildeter Duftstoffträger 68 aufgenommen ist. Das Gehäuse 66 weist ein Deckelteil 70 und ein Fußteil 72 auf, in welchen jeweils Durchgangsöffnungen 74 vorgesehen sind, die das Durchströmen des Gehäuses 66 und des Duftstoffträgers 68 mit Luft ermöglichen. Die Durchgangsöffnungen 74 sind vorzugsweise kleiner dimensioniert als die Partikel des Duftstoffträgers 68, so dass diese zuverlässig im Gehäuse 66 gehalten werden.

Das Deckelteil 70 und/oder das Fußteil 72 können dabei abnehmbar sein, so dass ein Nachfüllen des Duftstoffträgers 68 ermöglicht wird.

Auch in dieser Ausführungsform können die oben beschriebenen Kunststoffe, Duftstoffe und Zusatzstoffe Anwendung finden.

Insgesamt wird so ein Duftstoffspender erhalten, der auf ästhetisch ansprechende und platzsparende Weise montiert werden kann, besonders einfach in bestehende Automatisierungssysteme integriert werden kann und der eine besonders kontrollierte Freigabe des Duftstoffes bei besonders langer Lebensdauer ermöglicht.

## Patentansprüche

1. Duftstoffspender (20) zum Beduften eines Raumes, mit zumindest einem Duftstoffreservoir (28), welches in einem Gehäuse (22) aufgenommen ist, wobei das Gehäuse (22) als Unterputzgehäuse ausgebildet ist.

2. Duftstoffspender (20) nach Anspruch 1, bei welchem das Gehäuse (22) als Gerätedose, insbesondere als Zweifachgerätedose oder Dreifachgerätedose, nach DIN 49073 ausgebildet ist.

3. Duftstoffspender (20) nach Anspruch 1 oder 2, ferner umfassend einen Ventilator (26) in fluidischer Verbindung mit dem zumindest einen Duftstoffreservoir(28).

4. Duftstoffspender (20) nach Anspruch 3, ferner umfassend einen Gehäusedeckel (44) mit zumindest einer ersten Luftdurchtrittsöffnung (46), die den Ventilator (26) zumindest teilweise überdeckt, und zumindest einer zweiten Luftdurchtrittsöffnung (48), die das zumindest eine Duftstoffreservoir (28) zumindest teilweise überdeckt.

5. Duftstoffspender (20) nach einem der Ansprüche 1 bis 4, ferner umfassend ein Netzteil zum Bereitstellen einer Betriebsspannung, vorzugsweise einer Gleichspannung, von insbesondere 9 V bis 12 V für den Duftstoffspender (20).

6. Duftstoffspender (20) nach einem der Ansprüche 1 bis 5, ferner umfassend ein Steuergerät zum Ansteuern des Ventilators, welches dazu ausgelegt ist, zumindest einen Betriebsparameter des Duftstoffspenders (20), insbesondere eine Betriebszeit und/oder eine Laufgeschwindigkeit des Ventilators (26) und/oder eine Auswahl aus einer Mehrzahl von Duftstoffreservoirs (28) zu steuern.

7. Duftstoffspender (20) nach Anspruch 6, bei welchem das Steuergerät ein Eingabemodul umfasst, mittels welchem eine Steuervorgabe für den zumindest einen Betriebsparameter eingebbar ist.

8. Duftstoffspender (20) nach Anspruch 6 oder 7, bei welchem das Steuergerät ein Verbindungsmodul umfasst, mittels welchem Daten zwischen dem Steuergerät und einem Hausautomatisierungsbus austauschbar sind.

9. Duftstoffspender (20) nach einem der Ansprüche 6 bis 8, bei welchem das Steuergerät ein Kommunikationsmodul umfasst, mittels welchem Daten zwischen dem Steuergerät und einem Datenverarbeitungssystem außerhalb des Dufststoffspenders (20) drahtlos austauschbar sind.

10. Duftstoffspender (20) nach einem der Ansprüche 6 bis 9, bei welchem das Steuergerät ein Speichermodul umfasst, in welchem ein Steuerprogramm betreffend zumindest einen Betriebsparameter des Duftstoffspenders ablegbar ist.

11. Duftstoffspender (20) nach einem der Ansprüche 6 bis 10, bei welchem das Steuergerät einen Bewegungssensor und/oder Geräuschsensor umfasst und dazu ausgelegt ist, bei Feststellen einer Bewegung durch den Bewegungssensor und/oder eines Geräusches durch den Geräuschsensor den Ventilator für einen vorgegebenen Zeitraum zu aktivieren.

12. Duftstoffspender (20) nach einem der Ansprüche 1 bis 11, ferner umfassend eine Temperiervorrichtung zum Heizen und/oder Kühlen des zumindest einen Duftstoffreservoirs (28) .

13. Duftstoffspender (20) nach einem der Ansprüche 1 bis 12, bei welchem das zumindest eine Duftstoffreservoir (28) einen porösen, luftdurchlässigen Formkörper (56) aus einer Vielzahl von Kunststoffpartikeln umfasst, wobei die Kunststoffpartikel Duftstoffe umfassen.

14. Duftstoffspender (20) nach Anspruch 13, bei welchem der Formkörper (56) eine Mehrzahl von Lamellen (58) umfasst.

15. Duftstoffspender (20) nach Anspruch 14, bei welchem die Lamellen (58) zumindest teilweise mit Durchgangsöffnungen (60) versehen sind.

16. Duftstoffspender (20) nach Anspruch 13, bei welchem der Formkörper (56) eine Mehrzahl von miteinander verbundenen, insbesondere verschmolzenen, zumindest überwiegend kugelförmigen Kunststoffkörpern (64) umfasst.

17. Duftstoffspender (20) nach einem der Ansprüche 1 bis 16, bei welchem das zumindest eine Duftstoffreservoir (28) ein Gehäuse (66) mit einer Mehrzahl von Durchgangsöffnungen (74) umfasst, in welchem ein als Schüttgut ausgebildeter Duftstoffträger (68) aufgenommen ist.
